# EUROPÄISCHE PATENTANMELDUNG

(11) **EP 4 123 072 A1**
(43) Veröffentlichungstag der Anmeldung: **25.01.2023**
(21) Anmeldenummer: 21020379.0
(22) Anmeldetag: 22.07.2021
(51) Int. Cl.: D04B 1/26, D04B 1/12, D04B 1/18, A41B 11/00, A61F 13/08

(54) **KOMPRESSIVES GESTRICKTEIL**

(71) Anmelder: medi GmbH & Co. KG, 95448 Bayreuth (DE)
(72) Erfinder: Atmanspacher, Jan, 95485 Warmensteinach (DE)

(57) **Zusammenfassung**

Kompressives Gestrickteil (1, 1', 1", 1''') bestehend aus einem als Rundgestrick über mehrere Maschenreihen gestrickten Gestrickabschnitt (2, 2', 2", 2'''), der aus zumindest einen maschenbildenden Grundstrickfaden (3) sowie zumindest einen eingelegten und/ oder verstrickten elastischen Kompressionsfaden (4) gestrickt ist, wobei die Maschenköpfe (MK) und Maschenfüße (MF) der durch den zumindest einen Grundstrickfaden (3) gebildeten Grundstrickmaschen (GM) eine erste, insbesondere linke, Warenseite (5) und die Maschenschenkel (MS) eine zweite, insbesondere rechte, Warenseite (6) des Gestrickabschnitts (2, 2', 2", 2''') bilden und wobei der in die Grundstrickmaschen (GM) eingelegte und/ oder mit diesen verstrickte elastische Kompressionsfaden (4) an der ersten Warenseite des Gestrickabschnitts (2, 2', 2", 2''') zumindest stellenweise hervortritt und wobei die erste, insbesondere linke, Warenseite (5) des Gestrickabschnitts (2, 2', 2", 2''') im getragenen Zustand die Außenseite (7) und die zweite, insbesondere rechte, Warenseite (6) die Innenseite (8) des kompressiven Gestrickteils (1, 1', 1", 1''') bilden.

## Beschreibung

Die vorliegende Erfindung bezieht sich auf ein kompressives Gestrickteil nach dem Oberbegriff des Anspruch 1 sowie auf ein Verfahren zur Herstellung eines derartigen kompressiven Gestrickteils nach dem Oberbegriff des Anspruch 12.

Derartige kompressive Gestrickteile werden insbesondere als Arm- oder Beinbekleidungsstücke ausgebildet. Für den medizinischen Einsatz und/ oder für die Anwendung bei sportlichen Aktivitäten weisen diese oftmals einen oder mehrere kompressive Bereiche auf, welche dazu dienen, gezielt Druck auf den Körper eines Patienten auszuüben. Der auf den Körper eines Patienten ausgeübte Druck wird als Kompression bezeichnet. Ziel bei Kompressionsstrümpfen für den medizinischen Einsatz, beispielsweise in Form von Beinlingen mit oder ohne Hand- oder Fußteil, ist es unter anderem ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von Kompressionsstrümpfen in dem Sportbereich bewirken diese eine Leistungssteigerung bzw. eine verbesserte Regeneration.

Zur Ausbildung dieser kompressiven Gestrickteile werden diese vorzugsweise mittels einer Rundstrickmaschine rundgestrickt. Hierzu werden die Gestrickteile aus wenigstens einem maschenbildenden Grundstrickfaden gestrickt, in die ein elastischer Kompressionsfaden in jeder oder in jede x-ten Reihe eingelegt wird. Zur Herstellung, insbesondere zur Messung und Gütesicherung, von kompressiven Arm- oder Beinstrümpfen für die medizinische Anwendung existiert die RAL-GZ 387 der Gütezeichengemeinschaft. Aus den Prüfbestimmungen der RAL kann entnommen werden, wie bspw. der Druck eines Kompressionsstrumpfes auf ein Bein, zu bestimmen ist. Als Messmittel, insbesondere Kompressionsprüfgerät, wird die Prüfung am HOSY Messgerät (Institut Hohenstein) vorgeschlagen. Die Prüfung erfolgt anhand der Messung der Spannkraft in den mehreren Messpunkten, welche abhängig von der jeweiligen Dehnbarkeit des Gestricks, also abhängig von der Elastizität des Gestricks, variiert. Aus der Spannkraft wird die Kompression errechnet. D.h. je mehr Elastizität ein Gestrick in Gestrickquerrichtung aufweist, desto geringer ist die durch das Gestrick am Patienten ausgeübte Kompression. Andererseits je geringer die Elastizität des Gestrickteils ist, desto höher ist die am Patienten ausgeübte Kompression.

Aus dem Stand der Technik sind eine Vielzahl von, insbesondere kompressiven und rundgestrickten, Gestrickteilen, insbesondere in Form von Arm- oder Beinstrümpfen, bekannt. Ein vorzugsweise kompressives Rundgestrick, insbesondere in Form eines Beinbekleidungsstücks, ist beispielsweise aus der EP 3 733 941 A1 bekannt.

Dieses bekannte kompressive Rundgestrick, welches es zur Aufgabe hat, die Haftwirkung des Gestrickteils deutlich zu verbessern, besteht aus zumindest einen Grundstrickfaden, der über mehrere Maschenreihen hinweg Grundstrickmaschen bildet sowie zumindest einen eingelegten oder verstrickten elastischen Schussfaden, um dem Gestrick eine kompressive Wirkung zu verleihen. Damit das kompressive Gestrickteil darüber hinaus eine rutschhemmende Wirkung aufweist, ist auf die Grundstrickmaschen zumindest abschnittsweise, insbesondere im Bereich eines Bundes sowie im Fußsohlenbereich, ein Plüschhenkel ausbildender Plüschfaden aufplattiert, der ein Haftfaden ist.

Bei diesem kompressiven Gestrick handelt es sich, wie aus dem Stand der Technik allgemein bekannt ist und wie bereits zuvor erwähnt, um ein Rundgestrick, insbesondere um ein Rechts-Links-Gestrick, hergestellt auf einer Rundstrickmaschine. Bei einem Rechts-Links-Gestrick sind auf der rechten Warenseite nur rechte Maschen und auf der linken Seite nur linke Maschen sichtbar. D.h. die Maschenschenkel der Grundstrickmaschen bilden die eine, insbesondere rechte, Warenseite des Gestrickteils und die Maschenköpfe und Maschenfüße die andere, insbesondere die linke und dem Träger des Kleidungsstücks zugewandte, Warenseite des Gestrickteils.

Bei dieser aus dem Stand der Technik bekannten Lösung bilden ebenfalls die Maschenköpfe und Maschenfüße der Grundstrickmaschen die Innenseite des Beinbekleidungsstücks. Der überwiegende Teil des eingearbeiteten Silikonfadens, insbesondere die Maschenköpfe und Maschenfüße, ist nämlich in dem Gestrickinneren vorhanden, so dass folglich die Maschenschenkel die Außenseite des Gestrickteils bilden. Es handelt sich somit um ein klassisches Rechts-Links-Gestrick.

Aus dem Stand der Technik sind zudem eine Vielzahl von Hilfsmitteln bekannt, um die Anziehbarkeit derartiger kompressiver Kleidungsstücke, insbesondere von Strümpfen, zu erleichtern. Ein derartiges Hilfsmittel zum An- und Ausziehen von Strümpfen, insbesondere von Kompressionsstrümpfen, ist beispielsweise in der EP 1 791 453 B1 offenbart.

Hierbei besteht das Hilfsmittel aus einer reißfesten, friktionsarmen und sehr dünnen Kunststofffolie, die mit dem anzuziehenden Strumpf verwendet wird. So wird die Anziehbarkeit des kompressiven Strumpfes erleichtert. Das Hilfsmittel besteht konkret aus einem zum Ausziehen zu verwendenden Sockenteil sowie Zungenteil. Das Zungenteil wird hierbei auf etwa der Hälfte derart gefaltet, dass eine Doppellage besteht, wobei das freie Ende des doppellagigen Zungenteils als Aufnahme für den Fuß verwendet wird.

Als nachteilig der aus dem zuvor genannten Stand der Technik bekannten Ausgestaltung des kompressiven Gestrickteils erweist sich, dass das kompressive Gestrickteil selbst nur äußerst schwer anzuziehen ist, so dass weiterhin auf ein externes Hilfsmittel zur leichteren Anziehbarkeit zurückgegriffen werden muss. Das kompressive Gestrickteil selbst weist nämlich keine Eigenschaften zur leichteren Anziehbarkeit auf. Im Gegenteil, durch den eingearbeiteten, insbesondere in das Gestrick eingelegten, elastischen Schussfaden und der dadurch erzeugten kompressiven Drücke ist ein externes Hilfsmittel zur leichteren Anziehbarkeit unverzichtbar.

Es erweist sich auch als nachteilig, dass zur leichteren Anziehbarkeit mittels einer separaten Anziehhilfe diverse Unannehmlichkeiten verbunden sind. Zum einen muss die Anziehhilfe zusätzlich zu dem kompressiven Gestrickteil käuflich erworben werden, d.h. der Anwender hat einen finanziellen Aufwand. Zum anderen muss der Anwender die Anziehhilfe ständig zur Hand haben.

Schließlich erweist sich auch als nachteilig, dass derartige aus dem Stand der Technik bekannte Hilfsmittel meist nur das Anziehen der kompressiven Gestrickteile erleichtern, aber beim Ausziehen der Strümpfe keine Unterstützung ermöglichen, so dass die erschwerte Ausziehbarkeit weiterhin ein Problem bei Verwendung der aus dem Stand der Technik bekannten Lösungen darstellt.

Der vorliegenden Erfindung liegt daher die Aufgabe zugrunde ein kompressives Gestrickteil zu schaffen, welches die Nachteile aus dem Stand der Technik vermeidet, insbesondere die An- und Ausziehbarkeit eines derartigen kompressiven Gestrickteils deutlich erleichtert bzw. verbessert.

Gemäß einem Ausführungsbeispiel besteht das kompressive Gestrickteil aus einem als Rundgestrick über mehrere Maschenreihen gestrickten Gestrickabschnitt, der aus zumindest einen maschenbildenden Grundstrickfaden sowie zumindest einen eingelegten und/ oder verstrickten elastischen Kompressionsfaden gestrickt ist, wobei die Maschenköpfe und Maschenfüße der durch den zumindest einen Grundstrickfaden gebildeten Grundstrickmaschen eine erste, insbesondere linke, Warenseite und die Maschenschenkel eine zweite, insbesondere rechte, Warenseite des Gestrickabschnitts bilden und wobei der in die Grundstrickmaschen eingelegte und/ oder mit diesen verstrickte elastische Kompressionsfaden an der ersten Warenseite des Gestrickabschnitts zumindest stellenweise hervortritt und wobei die erste, insbesondere linke, Warenseite des Gestrickabschnitts im getragenen Zustand die Außenseite und die zweite, insbesondere rechte, Warenseite die Innenseite des kompressiven Gestrickteils bilden.

Bevorzugt ist der elastische Kompressionsfaden zumindest in jeder zweiten gestrickten Maschenreihe des kompressiven Gestrickteils in dieses eingearbeitet, besonders bevorzugt eingelegt bzw. hinterlegt. Hierdurch kommt der Kompressionsfaden im Bereich der Maschenköpfe einer Maschenreihe und den Maschenfüßen einer folgenden Maschenreihe zum Liegen.

Dadurch dass der elastische Kompressionsfaden, bevorzugt über weite Bereiche des Gestrickabschnitts, nicht mehr an der Innenseite des kompessiven Gestrickteils angeordnet ist, kommt es zu keiner erhöhten Reibung zwischen dem kompressiven Gestrickteil und der Haut eines Trägers erzeugt. Dies ermöglicht eine leichtere Anziehbarkeit des kompressiven Gestrickteils. Der elastische, insbesondere hochelastische, Kompressionsfaden, der an ersten Warenseite des Gestrickabschnitts zumindest stellenweise hervortritt, weist nämlich eine haftende Oberfläche auf oder ist aus einem gegenüber der Haut haftenden Material gefertigt, insbesondere aus Elastan, Gummi oder Kautschuk.

Gemäß einem zweiten Ausführungsbeispiel ist das obere und/ oder untere Ende des Gestrickabschnitts zur Bildung eines zweilagigen Bundes an der zweiten, insbesondere rechten, Warenseite des Gestrickabschnitts befestigt, so dass die erste, insbesondere linke, Warenseite des Gestrickabschnitts im Bereich des Bundes die Innenseite des kompressiven Gestrickteils bildet. Im Vergleich zu den aus dem Stand der Technik bekannten Lösungen, bei denen das Ende eines Gestrickabschnitts an der linken Warenseite des Gestrickteils befestigt ist, so dass die rechte Warenseite des kompressiven Gestrickteils im Bereich des Bundes die Innenseite des kompressiven Gestrickteils bildet, bildet nun die linke Warenseite des kompressiven Gestrickabschnitts im Bereich des Bundes die Innenseite. Dadurch, dass der eingearbeitete elastische Kompressionsfaden an der ersten, insbesondere linken, Warenseite des Gestrickabschnitts zumindest stellenweise hervortritt, ist es möglich der Innenseite des Bundes eine rutschhemmende Wirkung zu verleihen. Neben der leichteren Anziehbarkeit, dadurch dass der elastische Kompressionsfaden über weite Bereiche nicht mehr an der Innenseite des kompessiven Gestrickteils angeordnet ist und so keine erhöhte Reibung zwischen dem kompressiven Gestrickteil und der Haut eines Trägers erzeugt, ermöglicht das erfindungsgemäße Gestrickteil durch diese Ausbildung des Bundes gleichzeitig einen besseren Halt am Träger des kompressiven Gestrickteils.

Gemäß einem dritten Ausführungsbeispiel des kompressiven Gestrickteils ist das untere Ende des Gestrickabschnitts zur Bildung einer Zehenbox durch eine Naht, vorzugsweise durch eine flache Zehennaht, geschlossen, wobei die Naht zumindest teilweise auf der zweiten, insbesondere rechten, Warenseite des Gestrickabschnitts angeordnet ist. Dies bedeutet, dass zumindest deren Fadenenden, welche einen Teil der Naht darstellen, auf der rechten Warenseite, also auf der Innenseite des kompressiven Gestrickteils angeordnet sind. Die Naht ist hierbei bevorzugt als Überwendlich-Naht, als Rollnaht, oder als gekettelte Naht ausgebildet.

Gemäß einem weiteren Ausführungsbeispiel des kompressiven Gestrickteils ist in den Gestrickabschnitt zur Bildung einer Funktionszone, welche vorzugsweise zur Aufnahme einer Ferse eines Fußes ausgebildet ist, zumindest abschnittsweise zumindest ein zweiter maschenbildender Grundstrickfaden eingestrickt, wobei die Fadenenden des zumindest einen zweiten Grundstrickfadens auf der zweiten, insbesondere rechten, Warenseite des Gestrickabschnitts angeordnet sind. Die Funktionszone ist somit als stricktechnisch geformte Ferse, insbesondere durch eingestrickte Teilmaschenreihen, oder durch eine sogenannte Pendelferse, gebildet.

Gemäß einem weiteren Ausführungsbeispiel des kompressiven Gestrickteils ist auf den ersten Grundstrickfaden zumindest abschnittsweise ein erster Zusatzfaden derart aufplattiert, dass dessen Maschenschenkel in diesen Maschen die zweite, insbesondere rechte, Warenseite bilden. Bevorzugt ist der erste Zusatzfaden auf jeder x-ten Masche aufplattiert. Dazwischen ist dieser in den Grundstrickmaschen hinterlegt. Der erste Zusatzfaden bildet dabei zumindest abschnittsweise, insbesondere in Gestrickumfangsrichtung und Gestricklängsrichtung gesehen, die rechte Warenseite, insbesondere die Innenseite des Gestrickteils.

Gemäß einem weiteren Ausführungsbeispiel des kompressiven Gestrickteils ist der erste Zusatzfaden ein friktionsarmer Faden, insbesondere ein Polyamid, Polyester oder Polypropylenfaden, um dem kompressiven Gestrickteil an der zweiten, insbesondere rechten, Warenseite zumindest abschnittsweise eine leicht rutschende Wirkung zu verleihen. Hierdurch kann die Anziehbarkeit des kompressiven Gestrickteils zusätzlich verbessert werden, dies auf Grund der geringeren haftenden Eigenschaften der rechten Warenseite, insbesondere der Innenseite, des kompressiven Gestrickteils an der Haut eines Trägers durch das Aufplattieren des Zusatzfadens. Hierzu ist der friktionsarme Faden vorzugsweise möglichst vollflächig auf den Gestrickabschnitt aufplattiert.

Gemäß einem weiteren Ausführungsbeispiel des kompressiven Gestrickteils ist auf den ersten Grundstrickfaden zumindest abschnittsweise ein zweiter Zusatzfaden derart aufplattiert, dass dessen Maschenköpfe und Maschenfüße in diesen Maschen die erste, insbesondere linke, Warenseite bilden. Bevorzugt ist der zweite Zusatzfaden auf jeder x-ten Masche aufplattiert, dazwischen ist dieser in den Maschen hinterlegt. Der zweite Zusatzfaden bildet dabei die linke Warenseite des Gestrickteils, insbesondere die Außenseite des Gestrickteils. Dieser deckt somit bevorzugt den in die Grundstrickmaschen eingelegten und/ oder mit diesen verstrickten elastische Kompressionsfaden an der ersten Warenseite ab.

Der elastische Kompressionsfaden selbst besteht bevorzugt aus Elastan, Polyurethan, Polyether, Polyesterbasis, Gummi, Kautschuk, oder Silikon. Die Grundstrickfäden bestehen hingegen bevorzugt aus Polyamid, Polyester, Polypropylen oder einer Naturfaser. Der zweite Zusatzfaden ist bevorzugt ein Haftfaden, insbesondere Silikon-, Elastan- oder Kautschukfaden, um dem kompressiven Gestrickteil an der ersten, insbesondere linken, Warenseite zumindest abschnittsweise eine rutschhemmende Wirkung zu verleihen. Ist das kompressive Gestrickteil bspw. als Strumpf mit einem Fußteil ausgebildet, so ist der zweite Zusatzfaden bevorzugt im Bereich einer Fußsohle auf die Grundstrickmaschen des kompressiven Gestrickteils aufplattiert, um bspw. ein oder mehrere Grippelemente, insbesondere eine Antirutschsohle, zu bilden.

Gemäß einem weiteren Ausführungsbeispiel des kompressiven Gestrickteils sind der erste und/ oder zweite Zusatzfaden plüschhenkelbildend aufplattiert, so dass die durch die Zusatzfäden gebildeten Maschenköpfe an der ersten, insbesondere linken, Warenseite des Gestrickteils herausragen. Diese Plüschhenkel weisen bevorzugt unterschiedliche Höhen auf. Hierdurch kann bspw. eine Polsterung an der Außenseite des kompressiven Gestrickteils gebildet werden. Alternativ ist es auch denkbar, dass die Plüschhenkel ein Funktionselement für ein Verschlusselement, insbesondere eine Klettfläche für einen Klettverschluss, bilden. Ein zweites Verschlusselement mit Kletthaken ist vorzugsweise an der gebildeten Klettfläche anbringbar.

Das kompressive Gestrickteil nach den vorherigen Ausführungsbeispielen ist bevorzugt als Strumpf, insbesondere als Arm- oder Beinstrumpf, Socke, Beinling, Armling, Legging oder Hose, Bandage, insbesondere eine Fußbandage, Strumpfhose, oder als ein Gestrickteil einer Orthese ausgebildet. Besonders bevorzugt ist es als ein kompressives Beinbekleidungsstück ausgebildet, wobei die Kompressionsstärke in Gestricklängsrichtung, insbesondere von der Fessel in Richtung der Wadenmuskulatur, ab oder zunimmt. Die durch das Gestrickteil bzw. durch den Gestrickabschnitt erzeugten kompressiven Drücke betragen bevorzugt zwischen 5 und 60 mmHg, besonders bevorzugt zwischen 10 und 50 mmHg.

Gemäß einem Ausführungsbeispiel eines Verfahrens zum Herstellen eines kompressiven Gestrickteils weist dieses die Schritte:
- Rundstricken eines Gestrickabschnitts über mehrere Maschenreihen mit zumindest einen maschenbildenden Grundstrickfaden sowie zumindest einen eingelegten und/ oder verstrickten elastischen Kompressionsfaden, wobei die Maschenköpfe und Maschenfüße der durch den zumindest einen Grundstrickfaden gebildeten Grundstrickmaschen eine erste, insbesondere linke, Warenseite und die Maschenschenkel eine zweite, insbesondere rechte, Warenseite des Gestrickabschnitts bilden, und wobei der in die Grundstrickmaschen eingelegte und/ oder mit diesen verstrickte elastische Kompressionsfaden an der ersten Warenseite des Gestrickabschnitts zumindest stellenweise hervortritt und
   i. Umhängen und Befestigen des oberen und/ oder unteren Ende des Gestrickabschnitts an die zweite, insbesondere rechten, Warenseite des Gestrickabschnitts zur Bildung eines zweilagigen Bundes und/ oder
   ii. Schließen oder Schneiden und Schließen des unteren Ende des Gestrickabschnittsauf der zweiten, insbesondere rechten, Warenseite des Gestrickabschnitts zur Bildung einer geschlossenen Zehenbox und
- Wenden des rundgestrickten Gestrickabschnitts, so dass die erste, insbesondere linke, Warenseite des Gestrickabschnitts im getragenen Zustand die Außenseite und die zweite, insbesondere rechte, Warenseite die Innenseite des kompressiven Gestrickteils bilden.

Gemäß einem zweiten Ausführungsbeispiel eines Verfahrens zum Herstellen eines kompressiven Gestrickteils wird in Schritt a) zumindest ein erster Zusatzfaden auf der ersten, insbesondere linken, Warenseite und/ oder zumindest ein zweiter Zusatzfaden auf der zweiten, insbesondere rechten, Warenseite auf den zumindest einen maschenbildenden Grundstrickfaden aufplattiert. Bevorzugt wird der zumindest eine erste und/ oder zweite Zusatzfaden plüschhenkelbildend aufplattiert.

Gemäß einem weiteren Ausführungsbeispiel eines Verfahrens zum Herstellen eines kompressiven Gestrickteils wird in Schritt a) zumindest abschnittsweise zumindest ein zweiter maschenbildender Grundstrickfaden zur Bildung einer Funktionszone, welche vorzugsweise zur Aufnahme einer Ferse eines Fußes ausgebildet ist, eingestrickt.

Das vorliegende Beinbekleidungsstück zeichnet sich durch eine Reihe erheblicher Vorteile aus.

Durch die Ausbildung des kompressiven Gestrickteils derart, dass die erste, insbesondere linke, Warenseite des Gestrickabschnitts im getragenen Zustand die Außenseite und die zweite, insbesondere rechte, Warenseite die Innenseite des kompressiven Gestrickteils bilden, weist das Gestrickteil im Vergleich zu den aus dem Stand der Technik bekannten kompressiven Gestrickteilen eine deutlich verbesserte, insbesondere leichtere, Anziehbarkeit auf. Da der zur Erzeugung der kompressiven Wirkung eingearbeitete elastische Kompressionsfaden, der an der ersten, insbesondere linken, Warenseite des Gestrickteils stellenweise hervortritt sich nun beim Anlegen des kompressiven Gestrickteils, in einem getragenen Zustand des Getrickteils, als auch beim Ausziehen des Gestrickteils, im Wesentlichen an der Außenseite des Gestrickteils, insbesondere des kompressiven Kleidungsstücks, befindet, erzeugt dieser keine erhöhte Reibung zwischen dem kompressiven Gestrickteil und der Haut eines Trägers. Dieser besteht nämlich aus einem gegenüber der Haut eines Trägers haftendem Material, bspw. aus Elastan, Gummi oder Kautschuk. Folglich wird die haftende Eigenschaft, der dem Träger des kompressiven Gestrickteils zugewandte Warenseite des kompressiven Gestrickteils, erfindungsgemäß deutlich reduziert.

Besonders vorteilhaft ist, dass ferner durch die erfindungsgemäße stricktechnische Lösung neben einer leichteren Anziehbarkeit des kompressiven Gestrickteils gleichzeitig auch eine leichtere Ausziehbarkeit des kompressiven Gestrickteils ermöglicht wird. Es werden somit zwei Probleme, nämlich das erschwerte Anlegen und das erschwerte Ausziehen von kompressiven Kleidungsstücken, gelöst.

Einen weiteren Vorteil der Erfindung bildet die Möglichkeit des Verzichts auf eine externe An- und/ oder Ausziehhilfe zur erleichterten An- und/ oder Ausziehbarkeit des kompressiven Kleidungsstücks. Das Erfordernis ständig ein derartiges Hilfsmittel zur Hand zu haben entfällt. Zudem fallen auch keine zusätzlichen Kosten für eine derartiges Hilfsmittel an. Damit ist eine deutliche Kostenersparnis verbunden.

Auch der geringere Zeitaufwand beim Anlegen und Ausziehen des erfindungsgemäßen kompressiven Gestrickteils stellt einen klaren Vorteil gegenüber der aus dem Stand der Technik bekannten Lösungen dar. Das kompressive Gestrickteil muss nicht in oder an bzw. mit einem separaten Hilfsmittel positioniert bzw. kombiniert werden, damit das kompressive Kleidungsstück leichter an- oder auszuziehen ist.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des erfindungsgemäßen kompressiven Gestrickteils, ausgebildet als Beinbekleidungsstück, insbesondere als Beinstrumpf, in der Seitenansicht,
Figur 2 ein zweites Ausführungsbeispiel des erfindungsgemäßen kompressiven Gestrickteils, ausgebildet als Bandage, insbesondere als Fußbandage, ebenfalls in der Seitenansicht,
Figur 3 ein drittes Ausführungsbeispiel des erfindungsgemäßen kompessiven Gestrickteils, erneut ausgebildet als Bandage, insbesondere als Kniebandage,
Figur 4A ein Maschenbild, insbesondere die rechte und zweite Warenseite, eines ersten Ausführungsbeispiels des erfindungsgemäßen Gestrickabschnitts des kompressiven Gestrickteils, welche die Innenseite des Gestrickteils bildet,
Figur 4B ein Maschenbild, insbesondere die linke und erste Warenseite, des in Figur 4A gezeigten ersten Ausführungsbeispiels des erfindungsgemäßen Gestrickabschnitts des kompressiven Gestrickteils, welche die Außenseite des Gestrickteils bildet,
Figur 5A ein Maschenbild, insbesondere die rechte und zweite Warenseite, eines zweiten Ausführungsbeispiels des erfindungsgemäßen Gestrickabschnitts des kompressiven Gestrickteils, welche die Innenseite des Gestrickteils bildet,
Figur 5B ein Maschenbild, insbesondere die linke und erste Warenseite, des in Figur 5A gezeigten zweiten Ausführungsbeispiels des erfindungsgemäßen Gestrickabschnitts des kompressiven Gestrickteils, welche die Außenseite des Gestrickteils bildet,
Figur 6A ein Maschenbild, insbesondere die rechte und zweite Warenseite, eines dritten Ausführungsbeispiels des erfindungsgemäßen Gestrickabschnitts des kompressiven Gestrickteils, welche die Innenseite des Gestrickteils bildet,
Figur 6B ein Maschenbild, insbesondere die linke und erste Warenseite, des in Figur 6A gezeigten dritten Ausführungsbeispiels des erfindungsgemäßen Gestrickabschnitts des kompressiven Gestrickteils, welche die Außenseite des Gestrickteils bildet;
Figur 7A ein Maschenbild, insbesondere die rechte und zweite Warenseite, eines vierten Ausführungsbeispiels des erfindungsgemäßen Gestrickabschnitts des kompressiven Gestrickteils, welche die Innenseite des Gestrickteils bildet,
Figur 7B ein Maschenbild, insbesondere die linke und erste Warenseite, des in Figur 7A gezeigten dritten Ausführungsbeispiels des erfindungsgemäßen Gestrickabschnitts des kompressiven Gestrickteils, welche die Außenseite des Gestrickteils bildet;

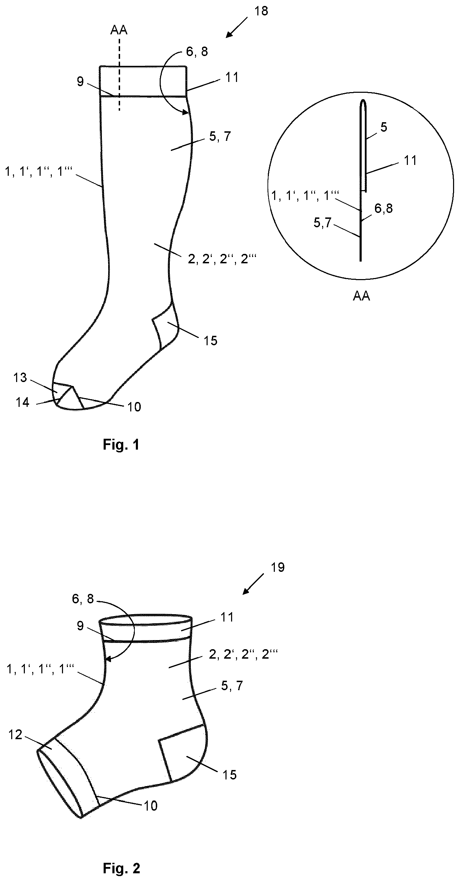

In Figur 1 ist ein erstes Ausführungsbeispiel des erfindungsgemäßen kompressiven Gestrickteils 1, 1', 1", 1''', ausgebildet als Beinbekleidungsstück, insbesondere als Beinstrumpf 18, in der Seitenansicht gezeigt. Ferner ist ein Querschnitt bzw. Ausschnitt AA des oberen Ende 9 des Beinstrumpfs 18 abgebildet. Der Beinstrumpf 18 besteht hierbei aus einem Gestrickabschnitt 2, 2', 2", 2''', der über mehrere Maschenreihen rundgestrickt ist und ein Rechts-Links-Gestrick bildet. Die Maschenköpfe und Maschenfüße der gebildeten Grundstrickmaschen bilden herbei eine erste, insbesondere linke, Warenseite 5 und die Maschenschenkel eine zweite, insbesondere rechte, Warenseite 6 des Gestrickabschnitts 2, 2', 2", 2'''. Zur Erzeugung von kompressiven Drücken ist in die Grundstrickmaschen ein elastischer Kompressionsfaden eingelegt und/ oder mit diesen verstrickt. Dieser tritt dadurch an der ersten Warenseite 5 des Gestrickabschnitts 2, 2', 2", 2''' zumindest stellenweise hervor. Erfindungsgemäß bildet nun die erste, insbesondere linke, Warenseite 5 des Gestrickabschnitts 2, 2', 2", 2''' die Außenseite 7 und die zweite, insbesondere rechte, Warenseite 6 die Innenseite 8 des kompressiven Gestrickteils 1, 1', 1", 1''' bzw. des Beinstrumpfes 18. Hierdurch ist der elastische Kompressionsfaden, bevorzugt über weite Bereiche des Gestrickabschnitts 2, 2', 2", 2''' nicht mehr an der Innenseite 8 des kompessiven Gestrickteils 1, 1', 1", 1''' angeordnet. Es kommt folglich zu keiner erhöhten Reibung zwischen dem kompressiven Gestrickteil 1, 1', 1", 1''', insbesondere zwischen dem elastischen Kompressionsfaden und der Haut eines Trägers. Dies ermöglicht eine leichtere Anziehbarkeit des kompressiven Gestrickteils 1, 1', 1", 1'''.

Zur Ausbildung des kompressiven Gestrickteils 1, 1', 1", 1''' als Beinstrumpf 18, weist das Gestrickteil 1, 1', 1", 1''' zumindest eine erste Funktionszone 15 auf, welche zur Aufnahme einer Ferse eines Fußes ausgebildet ist. Die Funktionszone 15 ist hierbei bevorzugt als stricktechnisch geformte Ferse, insbesondere durch eingestrickte Teilmaschenreihen mit Fadenanfang und Fadenende in jeder Reihe oder durch eine sogenannte Pendelferse, ausgebildet. An dem oberen Ende 9 weist der Beinstrumpf 18 zudem einen zweilagigen Bund 11 auf. Dieser ist durch den Gestrickabschnitt 2, 2', 2", 2''' selbst gebildet. Hierbei wird das obere Ende 9 des Gestrickabschnitts 2, 2', 2", 2''' auf die zweite, insbesondere rechte, Warenseite 6 des Gestrickabschnitts 2, 2', 2", 2''' umgehängt und dort befestigt. Dadurch bildet die erste, insbesondere linke, Warenseite 5 des Gestrickabschnitts 2, 2', 2", 2''' im Bereich des Bundes 11, also nur und ausschließlich im Bereich des Bundes 11, die Innenseite des kompressiven Gestrickteils 1, 1', 1", 1'''. In den übrigen Bereichen des kompressiven Gestrickteils 1, 1', 1", 1''' bildet die zweite, insbesondere rechte, Warenseite 6 die Innenseite 8 des Beinstrumpfes 18. Weil der eingearbeitete elastische Kompressionsfaden an der ersten, insbesondere linken, Warenseite 5 des Gestrickabschnitts 2, 2', 2", 2''' zumindest stellenweise hervortritt, verleiht dieser der Innenseite des Bundes 11 eine rutschhemmende Wirkung, wodurch ein besserer Halt des Beinstrumpfes 18 am Träger des kompressiven Gestrickteils 1, 1', 1", 1''' erzielt wird. Am unteren Ende 10 weist der Beinstrumpf 19 eine geschlossene Zehenbox 13 auf. Diese ist dadurch gebildet, dass der Gestrickabschnitt 2, 2', 2", 2''' des kompressiven Gestrickteils 1, 1', 1", 1''' durch eine Naht 14 geschlossen wird. Die Naht 14 ist hierbei zumindest teilweise auf der zweiten, insbesondere rechten, Warenseite 6 des Gestrickabschnitts 2, 2', 2", 2''' angeordnet. Dies bedeutet, dass zumindest die Fadenenden der Naht 14 auf der Innenseite 8 des kompressiven Gestrickteils 1, 1', 1", 1''' angeordnet sind.

Der Beinstrumpf 18 erzeugt hierbei bevorzugt kompressive Drücke in einem Fesselbereich bevorzugt zwischen 10 und 60 mmHg, und in einem Mittelfußbereich bevorzugt zwischen 5 und 50 mmHg. Das auf einer Rundstrickmaschine gestrickte kompressive Gestrickteil 1, 1', 1", 1''' weist hierzu einen oder mehrere in mehrere Grundstrickmaschenreihen eingearbeitete Kompressionsfäden bzw. Schussfäden auf. Die kompressiven Werte werden mittels der eingangs vorgestellten Messmethodik und Messgerät, insbesondere mittels der Prüfung am HOSY Messgerät (Institut Hohenstein), gemessen bzw. ermittelt.

Figur 2 zeigt ein zweites Ausführungsbeispiel des erfindungsgemäßen kompressiven Gestrickteils 1, 1', 1", 1''', ausgebildet als Bandage, insbesondere als Fußbandage 19. Die Fußbandage 19 besteht hierbei aus einem Gestrickabschnitt 2, 2', 2", 2''', der über mehrere Maschenreihen rundgestrickt ist. Hierbei ist der Gestrickabschnitt 2, 2', 2", 2''' ebenfalls durch eine Rechts-Links-Gestrick gebildet, in das zur Erzeugung kompressiver Drücke ein elastischer Kompressionsfaden eingearbeitet ist. Dabei bildet erfindungsgemäß, wie auch in Figur 1 beschrieben, die erste, insbesondere linke, Warenseite 5 des Gestrickabschnitts 2, 2', 2", 2''' die Außenseite 7 und die zweite, insbesondere rechte, Warenseite 6 die Innenseite 8 des kompressiven Gestrickteils 1, 1', 1", 1'''.

Zur Ausbildung des kompressiven Gestrickteils 1, 1', 1", 1''' als Fußbandage 19 ist in den Gestrickabschnitt 2, 2', 2", 2''' zumindest eine erste Funktionszone 15 eingestrickt, welche zur Aufnahme einer Ferse eines Fußes ausgebildet ist. Die stricktechnisch geformte Ferse ist erneut bevorzugt durch eingestrickte Teilmaschenreihen oder durch eine sogenannte Pendelferse gebildet. Zudem ist durch den Gestrickabschnitt 2, 2', 2", 2''' an dem oberen und untere Ende 9, 10 ein zweilagiger Bund 11, 12 ausgebildet. Hierzu ist, wie bereit in Figur 1 gezeigt und beschrieben, der Gestrickabschnitts 2, 2', 2", 2''' an der zweiten, insbesondere rechten, Warenseite 6 des Gestrickabschnitts 2, 2', 2", 2''' befestigt, so dass die erste, insbesondere linke, Warenseite 5 des Gestrickabschnitts 2, 2', 2", 2''' im Bereich des Bundes 11, 12 die Innenseite 8 des kompressiven Gestrickteils 1, 1', 1", 1''' bildet. Im restlichen kompressiven Gestrickteil 1, 1', 1", 1''' bildet die zweite, insbesondere rechte, Warenseite 6 des Gestrickabschnitts 2, 2', 2", 2''' die Innenseite 8 des kompressiven Gestrickteils 1,1', 1", 1'''.

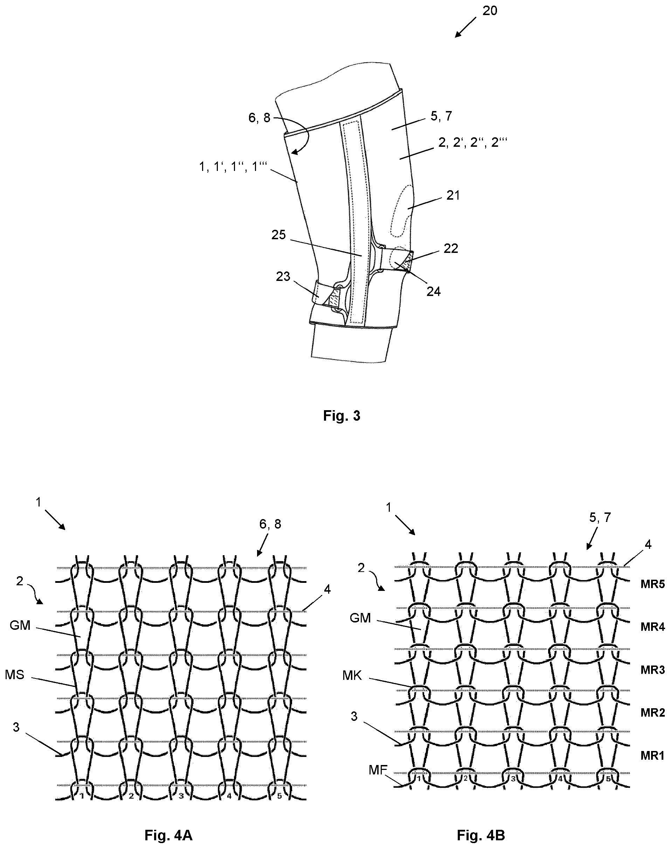

In Figur 3 ist nun ein drittes Ausführungsbeispiel des kompressiven Gestrickteils 1, 1', 1", 1''', ausgebildet als bzw. in Form einer Bandage, insbesondere Kniebandage 20, gezeigt. Die Kniebandage 20 umfasst hierbei ein rundgestricktes kompressives Gestrickteil 1, 1', 1", 1''' sowie ein in Tragestellung über die Patellasehne verlaufendes Funktionselement, nämlich eine Pelotte 21. Um ausreichend viel Druck auf die Sehne ausüben zu können, weist die Kniegelenkbandage 21 neben der bevorzugt lösbar am kompressiven Gestrickteil 1, 1', 1", 1''' ausgebildeten Pelotte 21 ferner bevorzugt die Spanngurte 22, 23 auf. Die Gurte 22, 23 sind hierbei bevorzugt über ein Verschlusssystem 24, bspw. in Form eines Klettverschlusses, in der Länge verstellbar. Zudem sind die Spanngurte 22, 23 besonders bevorzugt unlösbar mit dem kompressiven Gestrickteil 1, 1', 1", 1''' verbunden. D.h. diese sind an einem jeweils seitlich an dem kompressiven Gestrickteil 1, 1', 1", 1''' angeordnetem Stabilisierungselement 25, welches ebenfalls bevorzugt unlösbar mit dem kompressiven Gestrickteil 1, 1', 1", 1''' verbunden ist, fest angeordnet. Ein erster Spanngurt 22 verläuft hierbei vorderseitig nur abschnittsweise um das kompressive Gestrickteil 1, 1', 1", 1'''. Ein zweiter Spanngurt 23 ist rückseitig und höhenmäßig versetzt zum ersten Spanngurt 22 an der Kniebandage 20 vorgesehen. Auch dieser Gurt verläuft nur abschnittsweise um das kompressive Gestrickteil 1, 1', 1", 1'''.

Das kompressive Gestrickteil 1, 1', 1", 1''' selbst ist hierbei, wie auch in den Ausführungsbeispielen zuvor, als Rundgestrick ausgestaltet, welches über mehrere Maschenreihen gestrickt ist und dadurch einen Gestrickabschnitt 2, 2', 2", 2''' erzeugt. Dabei bildet erfindungsgemäß die erste, insbesondere linke, Warenseite 5 des Gestrickabschnitts 2, 2', 2", 2''' die Außenseite 7 und die zweite, insbesondere rechte, Warenseite 6 die Innenseite 8 des kompressiven Gestrickteils 1, 1', 1", 1'''.

Die Figuren 4A und 4B zeigen Maschenbilder eines ersten Ausführungsbeispiels des erfindungsgemäßen Gestrickabschnitts 2 des kompressiven Gestrickteils 1, insbesondere des Rechts-Links-Gestricks. Die Figur 4A zeigt dabei die rechte und zweite Warenseite 6 des Gestrickabschnitts 2, welche die Innenseite 8 des Gestrickteils 1 bildet. Die Figur 4B hingegen zeigt die linke und erste Warenseite 5, des Gestrickabschnitts 2, welche die Außenseite 7 des Gestrickteils 1 bildet. Die Maschenbilder zeigen den gestrickten Gestrickabschnitt 2, welcher aus einem Grundstrickfaden 3, der maschenbildend über mehrere Strickreihen MR1 bis MR5 und Maschenstäbchen M1 bis M5 hinweg verstrickt ist, gebildet ist. Bevorzugt besteht der Grundstrickfaden 3, der die Grundstrickmaschen GM bildet, aus Polyamid, Polyester, Polypropylen oder Polyurethan. Alternativ ist es möglich, dass dieser aus Naturfasern, insbesondere Wolle oder Zellulose bzw. Viskose, besteht. In die Grundstrickmaschen GM ist ein elastischer Kompressionsfaden 4 eingelegt. Der Kompressionsfaden 4 besteht hierbei bevorzugt aus Elastan, Polyurethan, Polyether, Polyesterbasis, Gummi, Kautschuk, oder Silikon. Der hochelastische Kompressionsfaden 4 ist in diesem Ausführungsbeispiel in jeder Maschenreihe MR1 bis MR5 eingelegt. Dies ist jedoch beliebig ausbildbar. Er kann auch nur in jeder zweiten Maschenreihe oder in jeder n-ten Maschenreihe eingelegt sein. Wie weiter in den Figuren 4A und 4B zu sehen ist, kommt der Kompressionsfaden 4 im Bereich der Maschenköpfe MK einer Maschenreihe, bspw. MR1, und den Maschenfüßen MF einer folgenden Maschenreihe, bspw. MR2, zum Liegen. Er ist hierbei 1:1 versetzt eingestrickt oder eingelegt.

In Figur 4A, in der die rechte Warenseite 6 des kompressiven Gestrickabschnitts 2, welche erfindungsgemäß die Innenseite 8 des Gestrickteils 1 bildet, gezeigt ist, bilden die Maschenschenkel MS der durch den Grundstrickfaden 3 gestrickten Grundstrickmaschen GM die Oberfläche der rechten Warenseite 6 und somit der Innenseite 8 des Gestrickteils 1. Die Maschenfüße MF und Maschenköpfe MK einer Maschenreihe MR kommen nämlich hinter den Maschenschenkeln MS einer vorherigen und nachfolgenden Maschenreihe MR zum Liegen. Da der elastische Kompressionsfaden 4 im Bereich der Maschenköpfe MK einer Maschenreihe und den Maschenfüßen MF einer folgenden Maschenreihe zum Liegen kommt, ist folglich auch dieser hinter den Maschenschenkeln MS der Grundstrickmaschen GM angeordnet. Somit ist die rechte Warenseite 6 ausschließlich durch die Maschenschenkel MS und durch den Grundstrickfaden 3 gebildet. Da der elastische Kompressionsfaden 4 nun nicht an der Innenseite 8 des kompessiven Gestrickteils 1 angeordnet ist, kommt es zu keiner erhöhten Reibung zwischen dem kompressiven Gestrickteil 1 und der Haut eines Trägers. Dies ermöglicht eine leichtere Anziehbarkeit des kompressiven Gestrickteils 1.

In Figur 4B ist nun die linke Warenseite 5 des Gestrickabschnitts 2, also die Außenseite 7 des Gestrickteils 1 gezeigt. Hierbei bilden die Maschenfüße MF und Maschenköpfe MK der durch den Grundstrickfaden 3 gestrickten Grundstrickmaschen GM die Oberfläche der linken Warenseite 7 und somit der Außenseite des Gestrickteils 1. Da der elastische Kompressionsfaden 4 im Bereich der Maschenköpfe MK, durch die stricktechnische Einbindung mit einem 1:1 Versatz, in jedem zweiten Maschenstäbchen vor den Maschenköpfen und Maschenfüßen zweier miteinander verstrickten Grundstrickmaschen GM zum Liegen kommt, tritt dieser zumindest stellenweise an der linken Warenseite 5 an deren Oberfläche hervor. Dadurch bildet auch dieser einen Teil der linken Warenseite 5 des Gestrickabschnitts 2. Da diese Seite 5 jedoch die Außenseite 7 des Gestrickteils 1 bildet, kann dieser auf Grund seiner technischen Eigenschaft keine erhöhte Reibung zwischen dem kompressiven Gestrickteil 1 und der Haut eines Trägers erzeugen.

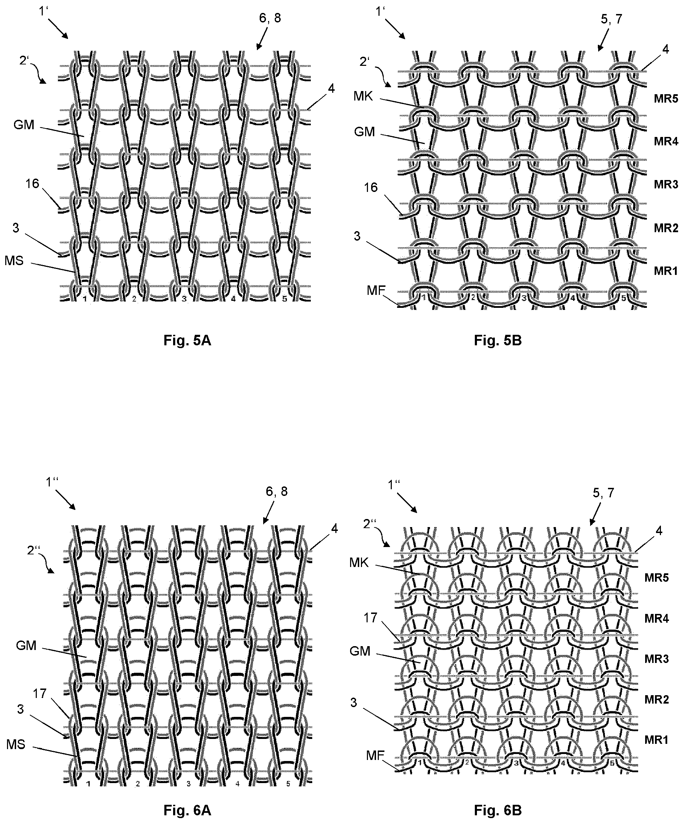

Die Figuren 5A und 5B zeigen Maschenbilder eines zweiten Ausführungsbeispiels des erfindungsgemäßen Gestrickabschnitts 1'. Die Figur 5A zeigt hierbei die rechte Warenseite 6 des Gestrickabschnitts 2', welche die Innenseite 8 des Gestrickteils 1' bildet. Figur 4B zeigt die linke Warenseite 7 des Gestrickabschnitts 2', welche die Außenseite 7 des Gestrickteils 1' bildet. Wie bereits in den Figuren 4A und 4B beschrieben, bildet auch in diesem Ausführungsbeispiel der Grundstrickfaden 3 über mehrere Maschenreihen MR1 bis MR5 und Maschenstäbchen M1 bis M5 hinweg mehrere Grundstrickmaschen GM. In die Grundstrickmaschen GM ist ebenfalls, wie in den Figuren 4A und 4B beschrieben, ein elastischer Kompressionsfaden 4 eingelegt.

Neben dem maschenbildend verstrickten Grundstrickfaden 3 und den eingelegten Schussfaden 4 umfasst der Gestrickabschnitt 2' des kompressiven Gestrickteils 1' nun ferner einen Zusatzfaden 16. Dieser ist auf den ersten Grundstrickfaden 3 aufplattiert und zwar derart, dass dessen Maschenschenkel MS die zweite, insbesondere rechte, Warenseite 6 des Gestrickabschnitts 2' bilden. Die Maschenschenkel MS des Zusatzfadens 16 bilden somit die Oberfläche der rechten Warenseite 6 und somit der Innenseite 8 des Gestrickteils 1'. Der Zusatzfaden 16 ist hierbei bevorzugt ein friktionsarmer Faden, insbesondere ein Polyamid, Polyester oder Polypropylenfaden, um dem kompressiven Gestrickteil 1' an der rechten Warenseite 6 zumindest abschnittsweise eine leicht rutschende Wirkung zu verleihen. Dies verbessert die Anziehbarkeit des kompressiven Gestrickteils 1' zusätzlich.

In Figur 5B ist nun die linke Warenseite 5 des Gestrickabschnitts 2', also die Außenseite 7 des Gestrickteils 1' zu sehen. Hierbei bilden die Maschenfüße MF und Maschenköpfe MK des Grundstrickfadens 3 die Oberfläche der linken Warenseite 5 und somit die Außenseite 7 des Gestrickteils 1'. Der elastische Kompressionsfaden 4 tritt zumindest stellenweise an der linken Warenseite 7 an der Oberfläche hervor, so dass auch dieser einen Teil der Oberfläche der Außenseite 7 des Gestrickteils 1' bildet.

In den Figuren 6A und 6B sind nun Maschenbilder eines dritten Ausführungsbeispiels des erfindungsgemäßen Gestrickabschnitts 1" zusehen, wobei die Figur 6A erneut die rechte Warenseite 6 des Gestrickabschnitts 2", welche die Innenseite 8 des Gestrickteils 1" bildet, und Figur 6B die linke Warenseite 5 des Gestrickabschnitts 2", welche die Außenseite 7 des Gestrickteils 1" bildet, gezeigt ist. Wie in den Ausführungsbeispielen und Figuren zuvor beschrieben, bildet auch in diesem Ausführungsbeispiel der Grundstrickfaden 3 über mehrere Maschenreihen MR1 bis MR5 und Maschenstäbchen M1 bis M5 hinweg mehrere Grundstrickmaschen GM. In die Grundstrickmaschen GM ist ebenfalls ein elastischer Kompressionsfaden 4 eingelegt.

In diesem Ausführungsbeispiel ist, wie insbesondere in Figur 6A gezeigt, nun neben dem maschenbildend verstrickten Grundstrickfaden 3 und den eingelegten Schussfaden 4 ein zweiter Zusatzfaden 17 vorgesehen. Dieser ist in diesem Ausführungsbeispiel auf den Grundstrickfaden 3 plüschhenkelbildend aufplattiert. Zudem ist dieser auf den Grundstrickfaden 3 bevorzugt derart aufplattiert, dass weiterhin die Maschenschenkel MS des Grundstrickfadens 3 die rechte Warenseite 6, insbesondere die Innenseite 8, des Gestrickabschnitts 2" bilden. Zeichnerisch ist in der Figur 6A der Zusatzfaden 17 neben dem Grundstrickfaden 3 abgebildet, dies jedoch lediglich zu verbesserten Darstellbarkeit. In Wirklichkeit sind die Maschenschenkel MS des Grundstrickfadens 3 vor den Maschenschenkeln des Zusatzfadens 17 angeordnet, so dass letztere sich im Gestrickinneren befinden und durch den Grundstrickfaden 3 abgedeckt sind. Alternativ ist es natürlich auch möglich, dass die Maschenschenkel MS des Zusatzfadens 17 die Innenseite 8 des Gestrickabschnitts 2" bilden.

Figur 6B zeigt nun die linke Warenseite 5 bzw. Außenseite 7 des Gestrickabschnitts 2" des kompressiven Gestrickteils 1'. Hierbei ragen die Maschenköpfe MK des zweiten Zusatzfadens 17 an der linken Warenseite 5, also der Außenseite 7 des Gestrickteils 1" heraus. Der zweite Zusatzfaden 17 ist bevorzugt ein Haftfaden, insbesondere Silikon-, Elastan- oder Kautschukfaden, um dem kompressiven Gestrickteil 1" an der Außenseite 7 zumindest abschnittsweise eine rutschhemmende Wirkung zu verleihen. Dies ermöglicht, neben der Ausbildung einer Polsterung, bspw. die Ausbildung von Grippelementen in einem Sohlenbereich eines kompressiven und gestrickten Strumpfes. Alternativ ist es denkbar, dass die durch den Zusatzfaden 17 gebildeten Plüschhenkel ein Funktionselement für ein Verschlusselement, insbesondere eine Klettfläche für einen Klettverschluss bilden. Ein zweites Verschlusselement mit Kletthaken ist hierbei bevorzugt an der gebildeten Klettfläche anbringbar.

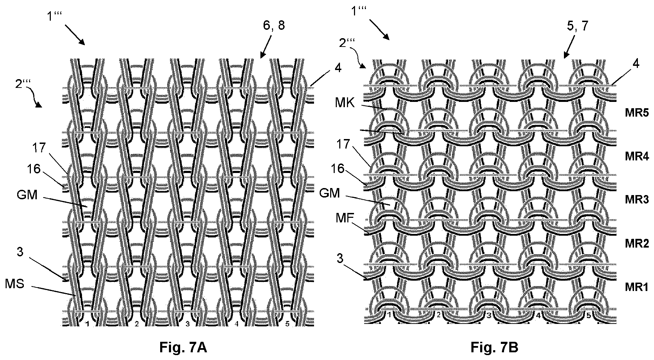

Schließlich wird in den Figuren 7A und Figur 7B ein viertes Ausführungsbeispiel des erfindungsgemäßen Gestrickabschnitts 1''' gezeigt. Die Figur 7A zeigt ebenfalls die rechte Warenseite 6 des Gestrickabschnitts 2''', also die Innenseite 8, und die Figur 7B die linke Warenseite 5 des Gestrickabschnitts 2''', welche die Außenseite 7 des Gestrickteils 1''' bildet. In diesem Ausführungsbeispiel sind sowohl der erste als auch der zweite Zusatzfaden 16, 17 gemäß den Beschreibungen zu den Figuren 5A und 5B sowie 6A und 6B neben dem elastischen Kompressionsfaden 4 in den Gestrickabschnitt 2''' des kompressiven Gestrickteils 1''' eingearbeitet. D.h. der erste Zusatzfaden 16 ist auf den Grundstrickfaden 3 derart aufplattiert, dass dessen Maschenschenkel MS die rechte Warenseite 6 und somit die Innenseite 8 des Gestrickteils 1''' bilden. Zudem ragen die Maschenköpfe MK des zweiten Zusatzfadens 17 an der linken Warenseite 5 des Gestrickteils 1" heraus, so dass diese im Wesentlichen die Außenseite 7 des Gestrickteils 1''' bilden.

Durch diese konkrete Ausgestaltung des Gestrickteil 1''' wird diesem, neben der kompressiven Wirkung, zum einen an der Innenseite 8, insbesondere bei Verwendung eines friktionsarmen Fadens als ersten Zusatzfaden 16, eine leicht rutschende Wirkung verliehen, was die Anziehbarkeit des kompressiven Gestrickteils 1''' wesentlich verbessert. Zum anderen weist das Gestrickteil 1''' an der Außenseite 7 durch die Ausbildung von Plüschhenkel eine polsternde Eigenschaft und/ oder eine rutschhemmende Wirkung auf, insbesondere bei Verwendung eines Haftfadens als zweiten Zusatzfaden 17. Darüber hinaus bildet das Gestrickteil 1''' selbst und direkt ein Funktionselement für ein Verschlusselement, insbesondere eine Klettfläche für einen Klettverschluss.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Kompressives Gestrickteil (1, 1', 1", 1''') bestehend aus einem als Rundgestrick über mehrere Maschenreihen gestrickten Gestrickabschnitt (2, 2', 2", 2"'), der aus zumindest einen maschenbildenden Grundstrickfaden (3) sowie zumindest einen eingelegten und/ oder verstrickten elastischen Kompressionsfaden (4) gestrickt ist, wobei die Maschenköpfe (MK) und Maschenfüße (MF) der durch den zumindest einen Grundstrickfaden (3) gebildeten Grundstrickmaschen (GM) eine erste, insbesondere linke, Warenseite (5) und die Maschenschenkel (MS) eine zweite, insbesondere rechte, Warenseite (6) des Gestrickabschnitts (2, 2', 2", 2''') bilden und wobei der in die Grundstrickmaschen (GM) eingelegte und/ oder mit diesen verstrickte elastische Kompressionsfaden (4) an der ersten Warenseite (5) des Gestrickabschnitts (2, 2', 2", 2''') zumindest stellenweise hervortritt, **dadurch gekennzeichnet, dass** die erste, insbesondere linke, Warenseite (5) des Gestrickabschnitts (2, 2', 2", 2''') im getragenen Zustand die Außenseite (7) und die zweite, insbesondere rechte, Warenseite (6) die Innenseite (8) des kompressiven Gestrickteils (1, 1', 1", 1''') bilden.

2. Kompressives Gestrickteil (1, 1', 1", 1''') nach Anspruch 1, **dadurch gekennzeichnet, dass** das obere und/ oder untere Ende (9, 10) des Gestrickabschnitts (2, 2', 2", 2''') zur Bildung eines zweilagigen Bundes (11, 12) an der zweiten, insbesondere rechten, Warenseite (6) des Gestrickabschnitts (2, 2', 2", 2''') befestigt ist, so dass die erste, insbesondere linke, Warenseite (5) des Gestrickabschnitts (2, 2', 2", 2''') im Bereich des Bundes (11, 12) die Innenseite (8) des kompressiven Gestrickteils (1, 1', 1", 1''') bildet.

3. Kompressives Gestrickteil (1, 1', 1", 1''') nach Anspruch 1 oder 2, **dadurch gekennzeichnet, dass** das untere Ende (10) des Gestrickabschnitts (2, 2', 2", 2''') zur Bildung einer Zehenbox (13) durch eine Naht (14) geschlossen ist, wobei die Naht (14) zumindest teilweise auf der zweiten, insbesondere rechten, Warenseite (6) des Gestrickabschnitts (2, 2', 2", 2''') angeordnet ist.

4. Kompressives Gestrickteil (1, 1', 1", 1''') nach einem der vorangehenden Ansprüche, **dadurch gekennzeichnet, dass** in den Gestrickabschnitt (2, 2', 2", 2''') zur Bildung einer Funktionszone (15), welche vorzugsweise zur Aufnahme einer Ferse eines Fußes ausgebildet ist, zumindest abschnittsweise zumindest ein zweiter maschenbildender Grundstrickfaden eingestrickt ist, wobei die Fadenenden des zumindest einen zweiten Grundstrickfadens auf der zweiten, insbesondere rechten, Warenseite (6) des Gestrickabschnitts (2, 2', 2", 2''') angeordnet sind.

5. Kompressives Gestrickteil (1', 1''') nach einem der vorherigen Ansprüche, **dadurch gekennzeichnet, dass** auf den ersten Grundstrickfaden (3) zumindest abschnittsweise ein erster Zusatzfaden (16) derart aufplattiert ist, dass dessen Maschenschenkel (MS) in diesen Maschen (GM) die zweite, insbesondere rechte, Warenseite (6) bilden.

6. Kompressives Gestrickteil (1', 1''') nach Anspruch 5, **dadurch gekennzeichnet, dass** der erste Zusatzfaden (16) ein friktionsarmer Faden, insbesondere ein Polyamid, Polyester oder Polypropylenfaden ist, um dem kompressiven Gestrickteil (1', 1''') an der zweiten, insbesondere rechten, Warenseite (6) zumindest abschnittsweise eine leicht rutschende Wirkung zu verleihen.

7. Kompressives Gestrickteil (1", 1''') nach einem der vorherigen Ansprüche, **dadurch gekennzeichnet, dass** auf den ersten Grundstrickfaden (3) zumindest abschnittsweise ein zweiter Zusatzfaden (17) derart aufplattiert ist, dass dessen Maschenköpfe (MK) und Maschenfüße (MF) in diesen Maschen (GM) die erste, insbesondere linke, Warenseite (5) bilden.

8. Kompressives Gestrickteil (1", 1''') nach Anspruch 7, **dadurch gekennzeichnet, dass** der zweite Zusatzfaden (17) ein Haftfaden, insbesondere Silikon-, Elastan- oder Kautschukfaden ist, um dem kompressiven Gestrickteil (1", 1''') an der ersten, insbesondere linken, Warenseite (5) zumindest abschnittsweise eine rutschhemmende Wirkung zu verleihen.

9. Kompressives Gestrickteil (1", 1''') nach einem der Ansprüche 5 bis 8, **dadurch gekennzeichnet, dass** der erste und/ oder zweite Zusatzfaden (17) plüschhenkelbildend aufplattiert sind, so dass die durch die Zusatzfäden (17) gebildeten Maschenköpfe (MK) an der ersten, insbesondere linken, Warenseite (5) des Gestrickteils (1", 1''') herausragen.

10. Kompressives Gestrickteil (1, 1', 1", 1''') nach einem der vorangehenden Ansprüche, **dadurch gekennzeichnet, dass** das Gestrickteil (1, 1', 1", 1''') ein Strumpf, insbesondere ein Arm- oder Beinstrumpf (18), eine Socke, eine Bandage (20), insbesondere eine Fußbandage (19), eine Strumpfhose, oder ein Gestrickteil (1, 1', 1", 1''') einer Orthese ist.

11. Kompressives Gestrickteil (1, 1', 1", 1''') nach Anspruch 10, **dadurch gekennzeichnet, dass** die durch den Gestrickabschnitt (2, 2', 2", 2''') erzeugten kompressiven Drücke zwischen 5 und 60 mmHg betragen.

12. Verfahren zum Herstellen eines kompressiven Gestrickteils (1, 1', 1", 1'''), **gekennzeichnet durch** die Schritte:
a) Rundstricken eines Gestrickabschnitts (2, 2', 2", 2''') über mehrere Maschenreihen (MR) mit zumindest einen maschenbildenden Grundstrickfaden (3) sowie zumindest einen eingelegten und/ oder verstrickten elastischen Kompressionsfaden (4), wobei die Maschenköpfe (MK) und Maschenfüße (MF) der durch den zumindest einen Grundstrickfaden (3) gebildeten Grundstrickmaschen (GM) eine erste, insbesondere linke, Warenseite (5) und die Maschenschenkel (MS) eine zweite, insbesondere rechte, Warenseite (6) des Gestrickabschnitts (2, 2', 2", 2''') bilden, und wobei der in die Grundstrickmaschen (GM) eingelegte und/ oder mit diesen verstrickte elastische Kompressionsfaden (4) an der ersten Warenseite (5) des Gestrickabschnitts (2, 2', 2", 2''') zumindest stellenweise hervortritt und
i. Umhängen und Befestigen des oberen und/ oder unteren Ende (9, 10) des Gestrickabschnitts (2, 2', 2", 2''') an die zweite, insbesondere rechten, Warenseite (6) des Gestrickabschnitts (2, 2', 2", 2''') zur Bildung eines zweilagigen Bundes (11, 12) und/ oder
ii. Schließen oder Schneiden und Schließen des unteren Ende (10) des Gestrickabschnitts (2, 2', 2", 2''') auf der zweiten, insbesondere rechten, Warenseite (6) des Gestrickabschnitts (2, 2', 2", 2''') zur Bildung einer geschlossenen Zehenbox (13) und
b) Wenden des rundgestrickten Gestrickabschnitts (2, 2', 2", 2'''), so dass die erste, insbesondere linke, Warenseite (5) des Gestrickabschnitts (2, 2', 2", 2''') im getragenen Zustand die Außenseite (7) und die zweite, insbesondere rechte, Warenseite (6) die Innenseite (8) des kompressiven Gestrickteils (1, 1', 1", 1''') bilden.

13. Verfahren zum Herstellen eines kompressiven Gestrickteils (1', 1", 1''') nach Anspruch 12, **dadurch gekennzeichnet, dass** in Schritt a) zumindest ein erster Zusatzfaden (16) auf der ersten, insbesondere linken, Warenseite (5) und/ oder zumindest ein zweiter Zusatzfaden (17) auf der zweiten, insbesondere rechten, Warenseite (6) auf den zumindest einen maschenbildenden Grundstrickfaden (3) aufplattiert wird.

14. Verfahren zum Herstellen eines kompressiven Gestrickteils (1", 1''') nach Anspruch 13, **dadurch gekennzeichnet, dass** der zumindest eine erste und/ oder zweite Zusatzfaden (17) plüschhenkelbildend aufplattiert wird.

15. Verfahren zum Herstellen eines kompressiven Gestrickteils (1, 1', 1", 1''') nach einem der Ansprüche 12 bis 14, **dadurch gekennzeichnet, dass** in Schritt a) zumindest abschnittsweise zumindest ein zweiter maschenbildender Grundstrickfaden zur Bildung einer Funktionszone (15), welche vorzugsweise zur Aufnahme einer Ferse eines Fußes ausgebildet ist, eingestrickt wird.
